# EUROPÄISCHE PATENTSCHRIFT

(11) **EP 1 127 864 B1**
(45) Veröffentlichungstag und Bekanntmachung des Hinweises auf die Patenterteilung: **19.11.2003**
(21) Anmeldenummer: 01102943.6
(22) Anmeldetag: 08.02.2001
(51) Int. Cl.: C07C 45/50, C07C 29/141

(54) **Verbessertes Verfahren zur Hydroformylierung von Olefinen durch Reduzierung der Ameisensäurekonzentration**
Improved process for the hydroformylation of olefins by reducing the concentration of formic acid
Procédé amélioré d' hydroformylation d' oléfines par réduction de la concentration de l' acide formique

(30) Priorität: 26.02.2000 DE 10009207
(43) Veröffentlichungstag der Anmeldung: 29.08.2001
(73) Patentinhaber: Oxeno Olefinchemie GmbH, 45772 Marl (DE)
(72) Erfinder: Kaizik, Alfred, Dr., 45772 Marl (DE); Tötsch, Walter, Dr., 45770 Marl (DE); Gosmann, Felix, 45701 Herten (DE); Büschken, Wilfried, Dr., 45721 Haltern (DE)

(56) Entgegenhaltungen:
- US-A- 3 935 285
- US-A- 5 237 105
- DATABASE WPI Section Ch, Week 199025 Derwent Publications Ltd., London, GB; Class E19, AN 1990-189703 XP002191398 & JP 02 124838 A (MITSUBISHI KASEI CORP), 14. Mai 1990 (1990-05-14)
- PATENT ABSTRACTS OF JAPAN vol. 1996, no. 09, 30. September 1996 (1996-09-30) & JP 08 117765 A (MITSUBISHI GAS CHEM CO INC), 14. Mai 1996 (1996-05-14)

## Beschreibung

Die Erfindung betrifft ein verbessertes Verfahren zur Hydroformylierung von Olefinen in Gegenwart von Kobaltcarbonylkomplexen durch Reduktion des Ameisensäuregehalts in der in den Hydroformylierungsreaktor zurückgeführten, Kobaltverbindungen enthaltenen wässrigen Lösung.

Höhere Alkohole, insbesondere solche mit 4 bis 25 Kohlenstoffatomen, können bekanntlich durch katalytische Hydroformylierung (oder Oxo-Reaktion) der um ein C-Atom kürzeren Olefine und anschließende katalytische Hydrierung der Aldehydund Alkohol-haltigen Reaktionsgemische hergestellt werden. Sie werden vorwiegend für die Herstellung von Weichmachern und Detergentien verwendet.

Die Art des Katalysatorsystems und die optimalen Reaktionsbedingungen für die Hydroformylierung sind von der Reaktivität des eingesetzten Olefins abhängig. Die Abhängigkeit der Reaktivität der Olefine von ihrer Struktur ist z.B. von J. Falbe, New Syntheses with Carbon Monoxide, Springer-Verlag, Berlin, Heidelberg, New York, 1980, Seite 95 ff. , beschrieben. Die unterschiedliche Reaktivität speziell der isomeren Octene ist ebenfalls bekannt (B. L. Haymore, A van Hasselt, R. Beck, Annals of the New York Acad.Sci., 415 (1983), Seiten 159-175).

Technische Olefingemische, die als Edukte für die Oxo-Synthese verwendet werden, enthalten Olefinisomere der verschiedensten Strukturen mit unterschiedlichen Verzweigungsgraden, unterschiedlicher Lage der Doppelbindung im Molekül und gegebenenfalls auch unterschiedlichen C-Zahlen. Dies gilt besonders für Olefingemische, die durch Di- oder Trimerisierung sowie weitergehende Oligomerisierung von C₂-C₅-Olefinen oder anderen leicht zugänglichen höheren Olefinen bzw. durch Cooligomerisierung der genannten Olefine entstanden sind. Als Beispiele für typische isomere Olefingemische, die durch Rhodium-katalysierte oder vorzugsweise durch Kobalt-katalysierte Hydroformylierung zu den entsprechenden Aldehyd- und Alkohol-Gemischen umgesetzt werden können, seien Tri- und Tetrapropene sowie Di- , Tri- und Tetrabutene genannt.

Sind Alkohole mit möglichst geringem Verzweigungsgrad als Hydroformylierungsprodukt angestrebt, wird die Hydroformylierung vorteilhaft mit unmodifizierten Kobaltkatalysatoren durchgeführt. Im Vergleich zu Rhodiumkatalysatoren werden mit Kobalt-Katalysatoren ausgehend von einem gleichen Olefingemisch höhere Ausbeuten an den besonders wertvollen geradkettigeren Oxo-Produkten erhalten.

Bei der Hydroformylierung von Olefinen unter der Katalyse von unmodifizierten Kobaltcarbonylkomplexen entstehen Reaktionsgemische, die Aldehyde, Alkohole, deren Ameisensäureester, nicht abreagierte Olefine, freie Ameisensäure und weitere Nebenprodukte sowie den Katalysator enthalten. Um ein nahezu Kobalt-freies Reaktionsgemisch zu erhalten, das für die Weiterverarbeitung geeignet ist, müssen die Kobaltverbindungen aus dem Hydroformylierungsaustrag abgetrennt werden. Außerdem müssen aus Kostengründen die abgetrennten Kobaltverbindungen in den Prozeß zurückgeführt werden.

Während der Hydroformylierung und/oder bei der Katalysatorabtrennung entsteht durch Hydrolyse von Ameisensäureestern Ameisensäure. Diese vermindern die Bildungsgeschwindigkeit des aktiven Katalysators, der Kobaltcarbonyle.

Ein konventionelles Verfahren zur Katalysator-Rückgewinnung aus dem Hydroformylierungsgemisch besteht darin, Kobaltverbindungen aus dem Reaktoraustrag mit wäßriger Base zu extrahieren, aus dem Extrakt durch Ansäuern HCo(CO)₄ freizusetzen und in die Hydroformylierung zurückzuführen (Kuhlmann-Verfahren). Nachteilig dabei ist, daß als Nebenprodukt ein Salz der eingesetzten Base entsteht, das entsorgt werden muß.

In GB 2055371 wird ein Verfahren beschrieben, daß folgende Stufen umfaßt:
a) Ein Teil der im Hydroformylierungsaustrag vorliegenden Kobaltcarbonyle wird mit einer wäßrigen Kobalt(II)salz-Lösung zu Co[Co(CO)₄]₂ umgesetzt. Dabei wird das komplexe Kobaltsalz in die Wasserphase extrahiert.
b) In einer zweiten Stufe werden die restlichen Kobaltcarbonyle in Gegenwart einer wäßrigen Säure mit Sauerstoff zu Kobalt(II)salzen oxidiert. Der dabei entstehende wäßrige Extrakt wird in der ersten Stufe verwendet.
c) Aus dem Extrakt der ersten Stufe werden durch Umsetzung mit Synthesegas in Gegenwart eines organischen Lösungsmittel die aktiven Kobaltverbindungen, d. h. der Kobaltkatalysator hergestellt.

Nachteilig hierbei ist, daß allein für die Abtrennung der Kobaltverbindungen aus dem Hydroformylierungsaustrag ein Oxidationschritt und zwei Phasentrennungen erforderlich sind.

In WO 93/24437 wird ein Verfahren zur Katalysatorabtrennung und -regenerierung beschrieben, das im Wesentlichen folgende Stufen umfasst:
a) Aus dem Hydroformylierungsaustrag werden nach Entspannen die Kobaltverbindungen unter reduktiven oder oxidativen Bedingungen (in Gegenwart von Sauerstoff) durch Extraktion mit einer wässrigen Lösung von maximal pH 7, insbesondere mit einer ameisensäurehaltigen, extrahiert.
   Bei der oxidativen Extraktion wird wie folgt aufgearbeitet:
b) Der wässrige Extrakt mit den Kobaltverbindungen wird destillativ aufkonzentriert. Als Kopfprodukt fällt ein Gemisch aus Wasser und Säuren (Ameisensäure) an, das zum Teil in die Extraktionsstufe a) zurückgeführt wird.
c) Das wässrige Konzentrat aus b) wird mit Synthesegas bei erhöhter Temperatur und erhöhtem Druck mit Synthesegas zu einem Gemisch mit Kobaltcarbonylen umgesetzt.
d) Aus dem aus c) erhaltenen Gemisch werden die Kobaltcarbonyle herausgestrippt. Der verbleibende wäßrige Strom wird in a) zurückgeführt.
e) Aus dem Strippgas werden die Kobaltcarbonyle mit Einsatzolefin (für die Hydroformylierung) extrahiert.

Bei der nicht-oxidativen Aufarbeitung werden die gleichen Verfahrensschritte, jedoch in anderer Reihenfolge (a - d - c - b) durchgeführt, mit dem weiteren Unterschied, daß der weitgehend entkobaltete wäßrige Strom aus Stufe d) in zwei Teilströme aufgetrennt wird, die in die Stufen a) und b) zurückgeführt werden.

WO 93/24438 und US 5 321 168 beschreiben Weiterentwicklungen des in WO 93/24437 offengelegten Verfahrens. Bei der gleichen Abfolge der Aufarbeitungsschritte wird bei der Katalysatorpräformierung (Stufe c) zusätzlich ein Palladiumkontakt verwendet. US 5 321 168 beschreibt zusätzlich die Wiederherstellung der Aktivität des Palladiumkontakts.

Diese Aufarbeitungsverfahren sind sehr komplex und erfordern ein hohes Investment und hohe Betriebskosten.

Ein weiteres Verfahren ist in J. Falbe, New Syntheses with Carbon Monoxide, Springer-Verlag, Berlin, Heidelberg, New York, 1980, Seite 164, 165 (BASF-Prozeß) beschrieben und besteht aus folgenden Stufen:
a) Oxidation der Kobaltcarbonyle mit Sauerstoff in Gegenwart von Säuren, insbesondere von im Prozeß anfallender Ameisensäure, zu Kobalt(II)salzen und deren Extraktion aus dem Hydroformylierungsgemisch
b) Reduktion der Kobalt(II)salze im wäßrigen Extrakt der Stufe a) in Gegenwart eines organischen Solvens zu Kobaltcarbonylen
c) Extraktion der Kobaltcarbonyle in eine organische Phase, zweckmäßig das Einsatzolefin, und deren Einspeisung in den Hydroformylierungsreaktor.

DE 196 54 340 beschreibt ein Hydroformylierungsverfahren, bei dem die Schritte b) und c) in situ im Hydroformylierungsreaktor durchgeführt werden, wodurch das Investment und die Betriebskosten gesenkt werden. Die Raum-Zeit-Ausbeuten dieses Verfahrens hängen auch von der Bildung und Stabilität des Katalysatorkomplexes im Hydroformylierungsreaktor ab. Dieses ist noch verbesserungsfähig.
In US 3 935 285 ist ein Verfahren zur Spaltung von Formiatestern im Nachgang einer Oxosynthese offenbart, bei dem die erhaltene Ameisensäure an Aluminiumoxid zersetzt wird. JP 02 124 838 schlägt zur Entfernung von Ameisensäure aus einem Hydroformylierungsprozess die destillative Abtrennung der Ameisensäure vor. Diese Verfahren sind für einen kontinuierlichen Hydroformylierungsprozess unter Rückführung des Kobaltkatalysators nicht geeignet.
Den bestehenden Verfahren ist gemeinsam, dass die Ameisensäurekonzentration entweder gar nicht beeinflusst bzw. kontrolliert wird und auf der sich durch die Reaktionsbedingungen einstellenden Konzentration bleibt, oder durch einen weiteren Verfahrensschritt wie eine Destillation abgesenkt wird.
Es bestand daher die Aufgabe, ein Hydroformylierungsverfahren für Olefine mit einem geschlossenen Kobalt-Katalysator-Kreislauf zu entwickeln, das neben einer einfachen Katalysatorrückführung eine hohe Raum-Zeit-Ausbeute ermöglicht.
Es wurde überraschenderweise gefunden, daß die Raum-Zeit-Ausbeute einer Hydroformylierungsreaktion gesteigert werden kann, wenn die Ameisensäurekonzentration in der wäßrigen Kobalt(II)salzlösung, die dem Hydroformylierungsreaktor zugeführt wird, bzw. im Gesamtprozeß gesenkt wird.
Gegenstand der vorliegenden Erfindung ist daher ein Verfahren zur Herstellung von Aldehyden mit 4 bis 25 Kohlenstoffatomen durch Hydroformylierung der entsprechenden Olefine in Gegenwart eines Kobaltkatalysators, wobei die bei der Hydroformylierung oder bei der anschließenden Katalysatoraufarbeitung gebildete Ameisensäure katalytisch zersetzt wird, indem das bei der Hydroformylierung gebildete Reaktionsgemisch in Gegenwart einer wäßrigen Phase durch oxidative Behandlung und anschließende Phasentrennung in eine organische Produktphase und eine wäßrige, Kobaltsalze und Ameisensäure enthaltende Phase getrennt, die wäßrige Phase ganz oder teilweise zur katalytischen Zersetzung der Ameisensäure eingesetzt und anschließend ganz oder teilweise in den Hydroformylierungsprozeß zurückgeführt wird.

Die mit den erfindungsgemäßen Verfahren hergestellten Rohaldehyde werden bevorzugt zur Herstellung von. Alkoholen durch Hydrierung verwendet.
Die Durchführung der Hydroformylierung und die Abtrennung des Kobaltkatalysators vom Hydroformylierungsaustrag kann z. B. wie in DE 196 54 340 A1 beschrieben, durchgeführt werden. Es handelt sich hier um einen 1-Stufen-Prozeß, bei dem die Olefine mit einer wäßrigen Kobaltsalzlösung und Synthesegas in homogener Phase, bevorzugt in einem kaskadierten Reaktor, besonders bevorzugt mittels einer Mischdüse umgesetzt werden. Der Reaktoraustrag wird anschließend oxidativ in Gegenwart einer wässrigen Lösung von Carbonsäuren, vorwiegend von Ameisensäure, behandelt; nach Phasentrennung wird ein Teil der wäßrigen, die Kobaltsalze enthaltenden Phase in den 1-Stufen-Prozeß zurückgeführt und die organische Phase zu den Produkten (Aldehyd) weiter aufgearbeitet oder zu den entsprechenden Alkoholen hydriert. Die in den Hydroformylierungsreaktor zurückgeführte wässrige Lösung enthält praktisch so viel Kobalt, wie mit dem Hydroformylierungsgemisch ausgetragen wird.
Zur katalytischen Zersetzung der Ameisensäure kann prinzipiell das bei der Hydroformylierung gebildete, Ameisensäure enthaltende Reaktionsgemisch vor oder nach der Oxidation der Kobaltcarbonyle eingesetzt werden. Die Abtrennung der Kobaltverbindungen vom Hydroformylierungsprodukt erfolgt erst danach. Dieses Vorgehen ist allerdings im Hinblick auf dem Gesamtprozess nicht ratsam.

Der Anteil der wässrigen Phase, der nicht zur Zersetzung der Ameisensäure eingesetzt wird, kann wieder in den Entkobaltungsschritt zurückgeführt werden.

Die so erhaltene, weitgehend von Ameisensäure befreite wässrige Lösung wird wieder in den Hydroformylierungsprozess zurückgeführt.

Es kann zur Reduzierung der Ameisensäure im Gesamtprozess ausreichend sein, die wässrige Phase, die zum Hydroformylierungsreaktor zurückgeführt werden soll, oder das Hydroformylierungsgemisch anteilig zur katalytischen Zersetzung der Ameisensäure einzusetzen. Ebenfalls ist es möglich im Zersetzungsreaktor nur einen Teil der Ameisensäure zu zersetzen. In den meisten Fällen genügt es, mindestens 50, bevorzugt 50 bis 80 % der im Prozess gebildeten Ameisensäure katalytisch zu zersetzen.

In jedem Fall enthält die zur Zersetzung der Ameisensäure eingesetzte Lösung oder Gemisch Kobaltverbindungen, sei es als Kobaltcarbonyl oder nach der oxydativen Aufarbeitung als Kobalt(II)salz. Die Konzentration dieser Kobaltverbindungen kann dabei in einem weiten Bereich variieren. Es muss nur sichergestellt sein, dass während der Aufarbeitung keine Kobaltverbindungen ausfallen. Die Konzentration der Kobaltsalzlösung ist durch die Wassermenge, die mit dem Hydroformylierungsgemisch aus dem Reaktor ausgetragen wird, mitbestimmt. Da mit dem entkobalteten Hydroformylierungsgemisch, d. h. der organischen Phase nach der Katalysatorabtrennung Wasser aus dem Prozeß ausgetragen wird, muss, um eine Aufkonzentrierung zu vermeiden, Wasser zudosiert werden. Mögliche Einspeisestellen hierzu sind beispielsweise der Reaktorzulauf, der Entkobalter oder der Rohproduktwäscher. Optional kann ein Teil der Ameisensäure destillativ abgetrennt werden und die Ameisensäure im Destillat nach dem erfindungsgmäßen Verfahren zersetzt werden.

Es ist bekannt, daß Ameisensäure mit Sauerstoff an Schwermetallkontakten, insbesondere an Pd-Kontakten, zu Kohlendioxid und Wasser umgesetzt wird. Ebenfalls kann Ameisensäure unkatalysiert, z.B. mit Wasserstoffperoxid, oxidiert werden.

Geeignete Katalysatoren für die Zersetzung der Ameisensäure im erfindungsgemäßen Verfahren sind Katalysatoren, die Al₂O₃, SiO₂, TiO₂, MgO sowie ihre Mischoxide, Zeolithe oder Aktivkohle als Trägermaterial enthalten. Als aktive Metalle können die im Verfahren verwendeten Katalysatoren Metalle aus den Gruppen 8 ,7b und 1b des Periodensystems der Elemente enthalten. Diese Metalle können in elementarer oder oxidischer Form, jeweils als Gemisch oder als Mischoxid eingesetzt werden. Im erfindungsgemäßen Verfahren werden bevorzugt Trägerkatalysatoren, insbesondere Pd/Al₂O₃-Kontakte, eingesetzt.

Bei der oxidativen Entfernung von Ameisensäure in wäßrigen Kobaltsalzlösungen wird Kobalt(II) teilweise zu Kobalt(III) oxidiert. Dies führt bei ansteigendem pH-Wert zu Ausfällungen von Co(III)-Verbindungen. Bei der Zersetzung unter Ausschluß von Sauerstoff oder eines anderen Oxidationsmittels findet diese Reaktion nicht statt. Erfindungsgemäß werden daher nicht oxidative Methoden bevorzugt, z. B. in einer Weise, dass die Zersetzung der Ameisensäure unter Anwesenheit von Synthesegas, Wasserstoff, Kohlenmonoxid, Kohlendioxid oder Stickstoff durchgeführt wird.

Inbesondere kann die Ameisensäure in der nach der oxidativen Entkobaltung des Hydroformylierungsaustrags anfallenden wäßrigen Kobaltsalzlösung katalytisch zersetzt werden.

Das erfindungsgemäße Verfahren zur Hydroformylierung unter Ameisensäure-Zersetzung kann kontinuierlich oder diskontinuierlich und sowohl in der flüssigen Phase und zumindestens partiell in der Gasphase durchgeführt werden. Die kontinuierliche Zersetzung in flüssiger Phase ist die bevorzugte Verfahrensweise.

Die Zersetzung der Ameisensäure kann in Rührreaktoren oder bevorzugt in Rohrreaktoren erfolgen. Im zweiten Fall kann im geradem Durchgang oder unter Lösungsrückführung (Schlaufe) gearbeitet werden. Die Reaktoren können als Gleichstromreaktoren mit Rieselbett (trickle flow) oder mit hohen Flüssigkeitsbelastungen (pulse flow) betrieben werden. Es können auch mehrere Reaktoren miteinander verschaltet werden. Diese können in gleicher oder unterschiedlicher Fahrweise betrieben werden.

Die Zersetzung der Ameisensäure erfolgt in einem Temperaturbereich von 100 °C bis 300 °C, vorzugsweise im Bereich 120 °C bis 200 °C. Der Gesamtdruck liegt in der Regel zwischen 1 bar und 350 bar, insbesondere zwischen 1 bar und 25 bar.

Die Katalysatorbelastungen bei der Ameisensäure-Zersetzung betragen 0,5 h⁻¹ bis 5 h⁻¹, insbesondere 1 h⁻¹ bis 3 h⁻¹.

Optional kann während der Ameisensäure-Zersetzung unter Anwesenheit eines Spülgases, wie beispielsweise Kohlenmonoxid, Stickstoff, Wasserstoff oder Synthesegas, durchgeführt werden.

Die an Ameisensäure verarmten bzw. von dieser befreiten wäßrigen Lösungen können gegebenenfalls nach Aufkonzentration, optional nach Vorvermischung mit Edukt und gegebenenfalls Synthesegas wieder in den Hydroformylierungsreaktor eingespeist werden.

In einer anderen Ausführungsform der vorliegenden Erfindung wird die katalytische Zersetzung der Ameisensäure in Gegenwart von Synthesegas unter Bildung des bei der Hydroformylierung der entsprechenden Olefine eingesetzten Kobaltkatalysators durchgeführt (sog. Präformierung).

Die Herstellung dieses Kobaltkatalysators (HCo(CO)₄ und Co₂(CO)₈) durch Umsetzung von wäßrigen Kobalt(II)- salzlösungen mit Synthesegas bei höheren Drücken und Temperaturen ist, wie bereits oben beschrieben, bekannt. Diese Reaktion kann mit Pd-Katalysatoren beschleunigt werden, wie es in den Patentschriften US 5 321 168 , US 5 434 318 und WO 93/24438 beschrieben ist. Dabei wird eine wäßrige Kobalt(II)salzlösung mit Synthesegas an einem Pd-Kontakt in Gegenwart eines organischen Lösungsmittel, vorzugsweise eines Alkohols, umgesetzt. Nachteilig bei diesen Verfahren ist, daß der Katalysator desaktiviert und in gewissen Zeitabständen regeneriert werden muß. Die Regenerierung erfolgt nach US 5 321 168 durch Spülen mit Wasser, optional unter Zusatz von Ameisensäure, unter Synthesegas bei einem Druck von 138-310 bar und einer Temperatur von 120-170°C. Nach US 5 434 318 wird die Spülung unter den oben genannten Bedingungen mit einem Gemisch aus Alkohol und wäßriger Ameisensäure vorgenommen.

Die genannten Verfahren umfassen nur die Bildung von Kobaltcarbonylen aus Kobalt(II)salzlösungen, nicht jedoch die Zersetzung von Ameisensäure.

In einer besonderen Ausführungsform der vorliegenden Erfindung kann die Zersetzung der Ameisensäure unter gleichzeitiger Bildung von Kobaltcarbonylverbindungen jeweils diskontinuierlich oder kontinuierlich durchgeführt werden.

Auch diese Umsetzung kann in Rührreaktoren oder bevorzugt in Rohrreaktoren erfolgen. Im zweiten Fall kann im geraden Durchgang oder unter Lösungsrückführung (Schlaufe) gearbeitet werden. Die Reaktoren können als Gleichstromreaktoren mit Rieselbett (trickle flow) oder mit hohen Flüssigkeitsbelastungen (pulse flow) betrieben werden. Es können auch mehrere Reaktoren miteinander verschaltet werden. Diese können in gleicher oder unterschiedlicher Fahrweise betrieben werden. Die kombinierten Umsetzungen können in Gegenwart von Synthesegas bei einem Gesamtdruck von 150 bar bis 330 bar, insbesondere zwischen 200 bar und 300 bar durchgeführt werden.

Im eingesetzten Synthesegas kann das molare Verhältnis zwischen Kohlenmonoxid und Wasserstoff im Bereich von 2/1 bis 1/2 liegen.

Die Reaktionstemperaturen können bei der kombinierten Ameisensäurezersetzung und Präformierung zwischen 140 °C und 220 °C, vorzugsweise zwischen 160 °C und 180 °C liegen. Die Katalatysatorbelastungen (LHSV) betragen 0,5 h⁻¹ bis 5 h⁻¹, vorzugsweise 1 h⁻¹ bis 3 h⁻¹.

Als Katalysatoren zur Zersetzung der Ameisensäure unter gleichzeitiger Bildung der Kobaltkatalysators können die gleichen verwendet werden, die bei der alleinigen Zersetzung der Ameisensäure eingesetzt werden.

Der flüssige Reaktoraustrag des kombinierten Zersetzungs- und Präformierungsreaktors wird in den Hydroformylierungsreaktor eingeleitet. Das anfallende Abgas wird in den Oxidator oder vorzugsweise ebenfalls in den Hydroformylierungsreaktor geleitet.

Optional kann die Zersetzung der Ameisensäure, gegebenenfalls unter gleichzeitiger Bildung der Kobaltcarbonyle, in Gegenwart organischer Lösungsmittel, die z. B. Alkohole oder Aldehyde, hier insbesondere die Edukte und Produkte der Hydroformylierungsreaktion, enthalten, durchgeführt werden. Zweckmäßig sind dies unter den Reaktionsbedingungen der Hydroformylierung flüssige Kohlenwasserstoffe und/oder die entsprechenden Olefine.

In jedem Fall bietet sich die Rückführung der bei der Zersetzung der Ameisensäure gebildeten Kobaltkatalysators in die Hydroformylierungsreaktion an.

Eine Hydroformylierungsanlage gemäß dem Verfahren der Erfindung erfordert im Vergleich zu einer konventionellen Anlage ein etwas höheres Investment. Dieser geringe Nachteil wird neben der höheren Raum-Zeit-Ausbeute durch weitere Vorteile überkompensiert:

Die Ameisensäurekonzentration in der Gesamtanlage, insbesondere im unteren Teil des Hydroformylierungsreaktors und im nachgeschalteten Oxidator (Entkobaltungseinrichtung), wird gesenkt.

Die Hydroformylierungsgeschwindigkeit und damit die Raum-Zeit-Ausbeute der Hydroformylierungsreaktion wird erhöht, da die mittlere Konzentration an aktiven Kobaltverbindungen im Hydroformylierungsreaktor größer ist.

Vorteilhaft ist weiterhin, daß durch die Zersetzung des größten Teils der im Prozeß erzeugten Ameisensäure nur Abströme anfallen, die weniger Ameisensäure enthalten. Dadurch wird die Umwelt entlastet.

Die folgenden Beispiele sollen die Erfindung weiter erläutern, nicht aber ihren Anwendungsbereich beschränken, der sich aus den Patentansprüchen ergibt.

### Beispiel 1 :

Ein Co-haltiges Prozeßwasser (3850 g) nach der Entkobaltung aus der Dibuten-Hydroformylierung, das neben dem Kobaltsalz (Co-Formiat) mit 0,85 Gew.-% Co (gerechnet als Co-Metall) und rd. 1,0 Gew.-% freie Ameisensäure enthielt, wurde in einer Kreislauf-Mitteldruckapparatur in Gegenwart von 198 g Pd/Al₂O₃-Trägerkatalysator (1.0 Gew.-% Pd,Al₂O₃-Strangextrudate 4 mm*4 mm) zwecks Reduzierung der Ameisensäuregehalte bei einer Temperatur von 135 °C und einem Druck von 15 bar behandelt. Durch gezielte Probenahme wurde die zeitliche Entwicklung der Kobalt- und Ameisensäuregehalte und des pH-Wertes im Co-Prozesswasser verfolgt. Die Edukt- und Produktanalysen gehen aus der folgenden Tabelle 1 hervor.

**Tabelle 1:**

| Zersetzung von Ameisensäure in Gegenwart von Pd/Al₂O₃-Katalysator | | | |
|---|---|---|---|
| Verweilzeit [min] | Ameisensäure [Gew.-%] | Kobalt-Gehalt [Gew.-%] | pH- Wert |
| 0 | 1,0 | 0,85 | 3,45 |
| 5 | 0,65 | 0,85 | 3,82 |
| 10 | 0,47 | 0,84 | 4,01 |
| 15 | 0,32 | 0,85 | 4,22 |
| 20 | 0,22 | 0,84 | 4,45 |
| 30 | 0,10 | 0,85 | 4,81 |

Man erkennt, daß die Gehalte an freier Ameisensäure im Co-Prozeßwasser in Gegenwart von Pd/Al₂O₃-Katalysator ohne Sauerstoff durch katalytische Zersetzung abgebaut werden können.

Die Abnahme des Ameisensäure-Anteils von 1,0 Gew.-% auf 0,10 Gew.-% hat eine Steigerung des pH-Wertes von 3,45 auf 4,81 zur Folge.

### Beispiel 2 :

In einem 2 Liter-Hochdruckautoklaven wurde das Co-haltige Prozeßwasser mit einer Zusammensetzung gemäß Beispiel 1 vor und nach der Ameisensäure-Zersetzung gemäß Beispiel 1 bei 180 °C und 270 bar mit Synthesegas (50 Vol.-% CO, 50 Vol.-% H₂) zwecks Herstellung von Kobaltcarbonylen, d. h. eines für die Olefin-Hydroformylierung aktiven Katalysatorsystems, carbonyliert. Das Co-Prozeßwasser enthielt 0,85 Gew.-% Co in Form von Co-Formiat und 1,0 bzw. 0,10 Gew.-% Ameisensäure. Nach einer Versuchszeit von 3 h wurde die Carbonylierung beendet und der Reaktionsaustrag auf den Co-Carbonyl-Gehalt hin untersucht. Zur Bestimmung des "aktiven" Kobalts im Co-Wasser wurde der Reaktionsaustrag mit Jod umgesetzt, um das CO aus den Co-Carbonylen freizusetzen. Aus den Mengen des freiwerdenden CO konnte die Konzentration der Co-Carbonyle und damit der Anteil des "aktiven" Kobalts in der Co-Gesamtmenge ermittelt werden.

Nach 3 h Versuchszeit weist das Ameisensäure-reiche Co-Wasser eine Aktivität von 21 % auf, d. h. von 0,85 Gew.-% Co liegt 21 % als Co-Carbonyl, d. h. in einer für die Hydroformylierung von Olefinen als Katalysator einsetzbaren Form vor. Demgegenüber wird beim Einsatz vom Co-Wasser mit geringem Ameisensäure-Gehalt nach der Ameisensäure-Zersetzung mit 38 % "aktiven" Kobalt deutlich höhere Aktivität erzielt.

### Beispiel 3 :

Ein Co-haltiges Prozesswasser aus der Entkobaltung einer Dibuten-Hydroformylierung mit 1,05 Gew.-% Co (gerechnet als Co-Metall) und rund 0,95 Gew.-% freie Ameisensäure wurde in Gegenwart von Pd/Al₂O₃-Trägerkatalysator (1.0 Gew.-% Pd) in Pulverforn in einem 2 Liter Hochdruckautoklaven (1 I Co-Wasser, 10 g Pd/Al₂O₃) bei 180 °C und 270 bar mit Synthesegas (50 Vol.-% CO, 50 Vol.-% H₂) zwecks Herstellung von Kobaltcarbonylen, d. h. eines für die Olefin-Hydroformylierung aktiven Katalysatorsystems, carbonyliert. Nach einer Versuchszeit von 3 h wurde die Umsetzung beendet und der Reaktionsaustrag wie im Beispiel 2 auf den Co-Carbonyl-Gehalt hin untersucht. Die durchgeführte Analyse des Reaktionsaustrages ergab eine Aktivität von rund 40 %.

Dieses Ergebnis zeigt, daß der Abbau der Ameisensäure-Gehalte durch Zersetzung und die Co-Carbonylierung in einem Verfahrenschritt durchgeführt werden kann.

### Beispiel 4 :

Das im Beispiel 2 carbonylierte Co-Wasser mit 0,85 Gew.-% Co (Gesamt Co gerechnet als Metall) und einer Co-Aktivität von 21 % wurde als Katalystor für die Hydroformylierung von Dibuten (C₈-Olefingemisch) zu C₉-Aldehyden verwendet. Hierfür wurden zuerst in einem 2 Liter - Hochdruckautoklaven die aktiven Co-Carbonyle aus dem Co-Wasser in das Edukt Dibuten ( 1000 g Dibuten, 250 g Co-Wasser ) unter Rühren extrahiert. Nach Abtrennen der wäßrigen Phase wurde das mit Co-Carbonylen beladene Dibuten bei einer Temperatur von 185 °C und bei einem Synthesegasdruck von 270 bar 5 Stunden lang hydroformyliert. Nach Abkühlung auf Raumtemperatur wurde das Reaktionsgemisch entspannt und durch Behandlung mit Essigsäure und Luft bei 80 °C vom Kobaltkatalysator befreit. Nach der durchgeführten GC-Analyse weist das Reaktionsgemisch folgende Zusammensetzung auf: 10,5 Gew.-% C₈-Olefine, 2,5 Gew.-% C₈-Paraffine, 54,8 Gew.-% C₉-Aldehyd, 22,6 Gew.-% C₉-Alkohol, 4,5 Gew.-% C₉-Formiate und 5,1 Gew.-% Hochsieder. Danach wurde ein Dibuten-Umsatz von 87,0 % bei einer Wertprodukt-Selektivität von 90,6 %, entsprechend einer Wertprodukt-Ausbeute von 78,8 % bezogen auf das eingesetzte Dibuten erhalten. Als Wertprodukte werden C₉-Aldehyde, C₉-Alkohole und C₉-Formiate betrachtet.

### Beispiel 5 :

Vergleichend zum Beispiel 4 wurde das im Beispiel 2 carbonylierte Co-Wasser mit einer erhöhten Co-Aktivität von 38 % als Katalystor für die Hydroformylierung von Dibuten zu C₉-Aldehyd verwendet. Hierfür wurden, wie im Beispiel 4 beschrieben, zuerst in einem 21- Hochdruckautoklaven die aktiven Co-Carbonyle aus dem Co-Wasser in das Edukt Dibuten (1000 g Dibuten, 250 g Co-Wasser) unter Rühren extrahiert. Nach Abtrennen der wäßrigen Phase wurde das mit Kobaltkatalysator beladene Dibuten bei 185 °C und 270 bar 5 Stunden lang hydroformyliert. Nach Abkühlung auf Raumtemperatur wurde das Reaktionsgemisch entspannt und durch Behandlung mit Essigsäure und Luft bei 80 °C vom Kobaltkatalysator befreit. Die GC-Analyse des Reaktionsgemisches nach der Entkobaltung weist folgende Zusammensetzung auf: 6,2 Gew.-% C₈-Olefine, 3,3 Gew.-% C₈-Paraffine, 55,1 Gew.-% C₉-Aldehyd, 25,4 Gew.-% C₉-Alkohol, 4,5 Gew.-% C₉-Formiate und 5,5 Gew.-% Hochsieder. Danach wurde ein Dibuten-Umsatz von 92,3 % bei einer Wertprodukt-Selektivität von 89,5 %, entsprechend einer Wertprodukt-Ausbeute von 82,6 % bezogen auf das eingesetzte Dibuten. Als Wertprodukte werden C₉-Aldehyde, C₉-Alkohole und C₉-Formiate betrachtet.

Man erkennt, daß eine Reduzierung der Ameisensäure-Gehalte im Co-Wasser zu einer deutlichen Verbesserung der Wertprodukt-Ausbeuten und damit der Raum-Zeit-Ausbeute der Reaktion führt.

## Patentansprüche

1. Verfahren zur Herstellung von Aldehyden mit 4 bis 25 Kohlenstoffatomen durch Hydroformylierung der entsprechenden Olefine in Gegenwart eines Kobaltkatalysators,
**dadurch gekennzeichnet,**
**daß** die bei der Hydroformylierung oder bei der anschließenden Katalysatoraufarbeitung gebildete Ameisensäure katalytisch zersetzt wird, indem das bei der Hydroformylierung gebildete Reaktionsgemisch in Gegenwart einer wäßrigen Phase durch oxidative Behandlung und anschließende Phasentrennung in eine organische Produktphase und eine wäßrige, Kobaltsalze und Ameisensäure enthaltende Phase getrennt, die wäßrige Phase ganz oder teilweise zur katalytischen Zersetzung der Ameisensäure eingesetzt und anschließend ganz oder teilweise in den Hydroformylierungsprozeß zurückgeführt wird.

2. Verfahren nach Anspruch 1,
**dadurch gekennzeichnet,**
**daß** zur katalytischen Zersetzung der Ameisensäure Katalysatoren, enthaltend Metalle der Gruppen 1b, 7b und 8 des Periodensystems der Elemente in elementarer oder oxidischer Form, jeweils als Gemisch oder als Mischoxid eingesetzt werden.

3. Verfahren nach Anspruch 2,
**dadurch gekennzeichnet,**
**daß** die Katalysatoren Al₂O₃, SiO₂, MgO, Zeolithe oder Aktivkohle als Tägermaterial enthalten.

4. Verfahren nach einem der Ansprüche 1 bis 3,
**dadurch gekennzeichnet,**
**daß** die katalytische Zersetzung der Ameisensäure kontinuierlich erfolgt.

5. Verfahren nach einem der Ansprüche 1 bis 4,
**dadurch gekennzeichnet,**
**daß** die katalytische Zersetzung der Ameisensäure bei einer Temperatur von 100 bis 300 °C und einem Druck von 1 bis 350 bar erfolgt.

6. Verfahren nach einem der Ansprüche 1 bis 5,
**dadurch gekennzeichnet,**
**daß** die katalytische Zersetzung der Ameisensäure zu mindestens 50 % erfolgt.

7. Verfahren nach einem der Ansprüche 1 bis 6,
**dadurch gekennzeichnet,**
**daß** die katalytische Zersetzung der Ameisensäure unter Anwesenheit von Synthesegas, Wasserstoff, Stickstoff oder Kohlenmonoxid durchgeführt wird.

8. Verfahren nach einem der Ansprüche 1 bis 7,
**dadurch gekennzeichnet,**
**daß** die katalytische Zersetzung der Ameisensäure in Gegenwart von Synthesegas unter Bildung des bei der Hydroformylierung der entsprechenden Olefine eingesetzten Kobaltkatalysators durchgeführt wird.

9. Verfahren nach einem der Ansprüche 1 bis 8,
**dadurch gekennzeichnet,**
**daß** die katalytische Zersetzung der Ameisensäure bei einem Druck von 150 bis 330 bar durchgeführt wird.

10. Verfahren nach einem der Ansprüche 8 oder 9,
**dadurch gekennzeichnet,**
**daß** die katalytische Zersetzung der Ameisensäure bei einer Temperatur von 140 bis 220 °C durchgeführt wird.

11. Verfahren nach einem der Ansprüche 1 bis 9,
daß die katalytische Zersetzung der Ameisensäure in Gegenwart organischer Lösungsmittel durchgeführt wird.

12. Verfahren nach Anspruch 11,
**dadurch gekennzeichnet,**
**daß** die organischen Lösungsmittel unter den Reaktionsbedingungen der Hydroformylierung flüssige Kohlenwasserstoffe und/oder die entsprechenden Olefine enthalten.

13. Verfahren nach Anspruch 11,
daß die organischen Lösungsmittel die aus der Hydroformylierung erhaltenen Aldehyde und/oder Alkohole enthält.

14. Verfahren nach einem der Ansprüche 8 bis 13,
**dadurch gekennzeichnet,**
**daß** der bei der katalytischen Zersetzung der Ameisensäure gebildete Kobaltkatalysator in die Hydroformylierungsreaktion zurückgeführt wird.

## Claims

1. A process for preparing aldehydes having from 4 to 25 carbon atoms by hydroformylation of the corresponding olefins in the presence of a cobalt catalyst, **characterized in that** the formic acid formed in the hydroformylation or in the subsequent catalyst work-up is decomposed catalytically by subjecting the reaction mixture formed in the hydroformylation to an oxidative treatment in the presence of an aqueous phase and subsequently separating the phases to give an organic product phase and an aqueous phase comprising cobalt salts and formic acid, using all or part of the aqueous phase for the catalytic decomposition of formic acid and subsequently returning all or part of it to the hydroformylation process.

2. A process according to claim 1, **characterized in that** catalysts comprising metals of groups Ib, VIIa and VIII of the Periodic Table of the Elements are used in elemental or oxidic form, in each case as a mixture or as a mixed oxide, for the catalytic decomposition of formic acid.

3. A process according to claim 2, **characterized in that** the catalysts comprise Al₂O₃, SiO₂, MgO, zeolites or activated carbon as support material.

4. A process according to any one of claims 1 to 3, **characterized in that** the catalytic decomposition of formic acid is carried out continuously.

5. A process according to any one of claims 1 to 4, **characterized in that** the catalytic decomposition of formic acid is carried out at a temperature of from 100 to 300°C and a pressure of from 1 to 350 bar.

6. A process according to any one of claims 1 to 5, **characterized in that** the catalytic decomposition of the formic acid proceeds to an extent of at least 50%.

7. A process according to any one of claims 1 to 6, **characterized in that** the catalytic decomposition of formic acid is carried out in the presence of synthesis gas, hydrogen, nitrogen or carbon monoxide.

8. A process according to any one of claims 1 to 7, **characterized in that** the catalytic decomposition of formic acid is carried out in the presence of synthesis gas to form the cobalt catalyst used in the hydroformylation of the respective olefins.

9. A process according to any one of claims 1 to 8, **characterized in that** the catalytic decomposition of formic acid is carried out at a pressure of from 150 to 330 bar.

10. A process according to either of claims 8 or 9, **characterised in that** the catalytic decomposition of formic acid is carried out at a temperature of from 140 to 220°C.

11. A process according to any one of claims 1 to 9, **characterized in that** the catalytic decomposition of formic acid is carried out in the presence of organic solvents.

12. A process according to claim 11, **characterized in that** the organic solvents comprise hydrocarbons which are liquid under the reaction conditions of the hydroformylation and/or the respective olefins.

13. A process according to claim 11, **characterized in that** the organic solvents comprise the aldehydes and/or alcohols obtained from the hydroformylation.

14. A process according to any one of claims 8 to 13, **characterized in that** the cobalt catalyst formed in the catalytic decomposition of formic acid is recirculated to the hydroformylation reaction.

## Revendications

1. Procédé de préparation d'aldéhydes ayant de 4 à 25 atomes de carbone, par hydroformylation des oléfines correspondantes en présence d'un catalyseur au cobalt,
**caractérisé en ce que**
l'acide formique formé au cours de l'hydroformylation ou lors de la purification consécutive du catalyseur, est décomposé, procédé dans lequel le mélange réactionnel formé au cours de l'hydroformylation est en présence d'une phase aqueuse par traitement oxydatif et séparation de phase consécutive, séparé en une phase de produit organique et une phase aqueuse contenant des sels de cobalt et de l'acide formique, la phase aqueuse est utilisée totalement ou partiellement en vue de la décomposition catalytique de l'acide formique et est ensuite recyclée totalement ou partiellement dans le processus d'hydroformylation.

2. Procédé selon la revendication 1,
**caractérisé en ce qu'**
on met en oeuvre en vue de la décomposition catalytique de l'acide formique, les catalyseurs contenant des métaux des groupes 1b, 7b et 8 du système périodique des éléments sous forme élémentaire ou oxydique, respectivement en tant que mélange ou en tant qu'oxyde mixte.

3. Procédé selon la revendication 2,
**caractérisé en ce que**
les catalyseurs contiennent Al₂O₃, SiO₂, MgO, des zéolithes, ou du charbon actif, comme matériau de support.

4. Procédé selon l'une des revendications 1 à 3,
**caractérisé en ce que**
la décomposition catalytique de l'acide formique s'effectue en continu.

5. Procédé selon l'une des revendications 1 à 4,
**caractérisé en ce que**
la décomposition catalytique de l'acide formique s'effectue à une température de 100 à 300°C et à une pression de 1 à 350 bars.

6. Procédé selon l'une des revendications 1 à 5,
**caractérisé en ce que**
la décomposition catalytique de l'acide formique s'effectue pour au moins 50 %.

7. Procédé selon l'une des revendications 1 à 6,
**caractérisé en ce que**
la décomposition catalytique de l'acide formique est effectuée en présence de gaz de synthèse, d'hydrogène, d'azote ou de monoxyde de carbone.

8. Procédé selon l'une des revendications 1 à 7,
**caractérisé en ce que**
la décomposition catalytique de l'acide formique est effectuée en présence de gaz de synthèse avec formation du catalyseur au cobalt mis en oeuvre au cours de l'hydroformylation de l'oléfine correspondante.

9. Procédé selon l'une des revendications 1 à 8,
**caractérisé en ce que**
la décomposition catalytique de l'acide formique est effectuée à une pression de 150 à 330 bars.

10. Procédé selon l'une des revendications 8 ou 9,
**caractérisé en ce que**
la décomposition catalytique de l'acide formique est effectuée à une température de 140 à 220°C.

11. Procédé selon l'une des revendications 1 à 9,
**caractérisé en ce que**
la décomposition catalytique de l'acide formique est effectuée en présence de solvants organiques.

12. Procédé selon la revendication 11,
**caractérisé en ce que**
les solvants organiques renferment dans les conditions de la réaction d'hydroformylation, des hydrocarbures liquides et/ou les oléfines correspondantes.

13. Procédé selon la revendication 11,
**caractérisé en ce que**
le solvant organique contient les aldéhydes et/ou les alcools obtenus à partir de l'hydroformylation.

14. Procédé selon l'une des revendications 8 à 13,
**caractérisé en ce que**
le catalyseur au cobalt, formé au cours de la décomposition catalytique de l'acide formique, est recyclé dans la réaction d'hydroformylation.
